# EUROPEAN PATENT SPECIFICATION

(11) **EP 4 023 168 B1**
(45) Date of publication and mention of the grant of the patent: **31.07.2024**
(21) Application number: 22158936.9
(22) Date of filing: 22.08.2018
(51) Int. Cl.: A61B 17/072, A61B 90/00, A61B 17/00

(54) **CONTACTLESS LOADING UNIT DETECTION**
KONTAKTLOSER LADEVORRICHTUNGSNACHWEIS
DÉTECTION D'UNITÉ DE CHARGEMENT SANS CONTACT

(30) Priority: 23.08.2017 US 201762549294 P; 24.07.2018 US 201816043230
(43) Date of publication of application: 06.07.2022
(62) Divisional of application: 18190152.1
(73) Proprietor: Covidien LP, Mansfield, MA 02048 (US)
(72) Inventor: PANTAZIS, John, Stratford, CT 06614 (US); CALDERONI, Anthony, Bristol, CT 06010 (US)
(74) Representative: Maschio & Soames IP Ltd

(56) References cited:
- EP-A1- 3 064 146
- EP-A2- 0 189 807
- EP-A2- 2 923 653
- EP-A2- 2 992 839
- JP-A- 2016 011 864
- US-A1- 2011 017 801

## Description

### CROSS-REFERENCE TO RELATED APPLICATIONS

This application claims the benefit of and priority to U.S. Provisional Patent Application Serial No. 62/549,294 filed August 23, 2017.

### BACKGROUND

### 1. Technical Field

The present disclosure relates to surgical instruments and, more specifically, to surgical instruments having contactless detection systems for determining connection between components of the surgical instrument.

### 2. Discussion of Related Art

Surgical instruments having a handle and a loading unit releasably coupled to the handle are known. Generally, the loading unit receives mechanical input from the handle to actuate a tool of the loading unit.

Some surgical instruments include a detection system to identify and notify a clinician when the loading unit is properly coupled to the handle. Generally, the detection system includes contacts and a mechanical switch. In such instruments, the contacts may be exposed to bodily fluids, e.g., blood or saline. This fluid exposure may result in malfunctioning of the detection system.

In addition, surgical instruments that include detection systems having exposed contacts may be damaged during resterilazation of the surgical instruments. More specifically, during an autoclave process, exposed electrical contacts may be susceptible to damage from the steam and the high-pressure fluids used in the autoclave process. For example, during the autoclave process the exposed electrical contacts may corrode, form dendtric growths, or electro-plate.

Accordingly, a continuing need exists for detection systems that are not susceptible fluid ingress and/or damage from a resterilization process.

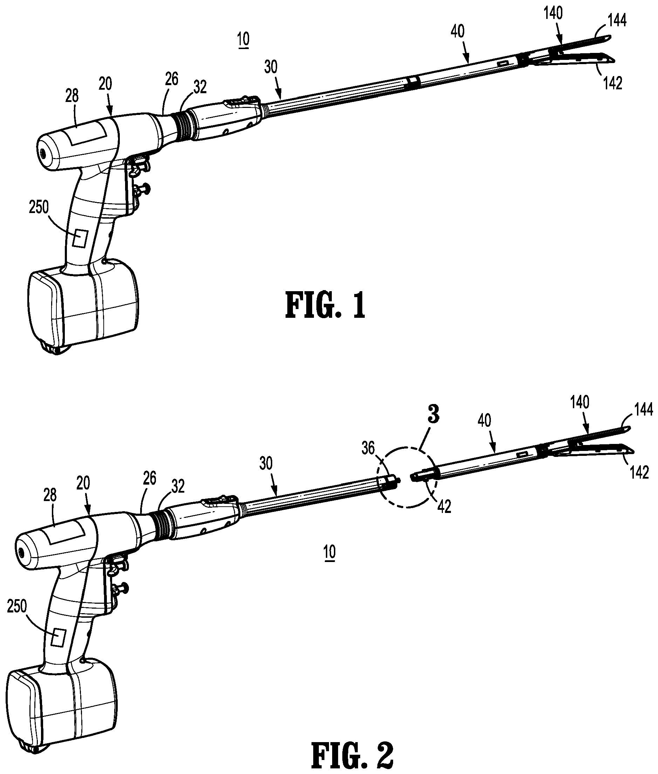

EP 2 923 653 discloses a surgical instrument with magnetic detection means to ensure proper connection of a loading unit (a fastener cartridge ) to an adapter (see figures 1 and 62). A sensor is provided in the cartridge, and a magnet is provided in the jaw receiving this cartridge.

### SUMMARY

The invention is defined in the appended claims.

In an aspect of the present disclosure, a loading unit detection system includes an elongate member, a loading unit, a magnet, and a giant magneto-resistance integrated circuit ("GMR IC"). The elongate member defines a receiver. The loading unit includes a connector that is configured to be received within the receiver to releasably couple the loading unit to the elongate member. The magnet is supported on the connector and is configured to translate relative to the elongate member as the connector is received within the receiver. The GMR IC is embedded within the elongate member and is configured to output a differential voltage in response to a magnetic field produced by the magnet of the loading unit. The differential voltage is indicative of the position of the loading unit within the receiver of the elongate member. The magnet may be embedded within the connector of the loading unit.

In aspects, the loading unit detection system includes a secondary magnet that is embedded within the loading unit such that the differential voltage of the GMR IC induced by a combined magnetic field of the magnet and the secondary magnet is indicative of the position of the loading unit within the receiver of the elongate member and the type of the loading unit.

In some aspects, the loading unit detection system includes an operational amplifier and a latch. The operational amplifier may receive the differential voltage from the GMR IC and transmit an amplified output to the latch. The latch may have a first state when the amplified output is below a threshold and a second state when the amplified output is above the threshold. The amplified output may be below the threshold when the connector is in an uncoupled state with the receiver and above the threshold when the receiver is in a coupled state with the receiver. The operation amplifier and the latch may be integrated with the GMR IC.

In certain aspects, the loading unit detection system includes an instrument amplifier and a filter. The instrument amplifier receives the differential voltage from the GMR IC and outputs an amplified signal to the filter. The filter may be configured to output a filtered signal indicative of the position of the connector relative to the receiver and a type of the loading unit.

In another aspect of the present disclosure, a surgical instrument includes a handle, an adapter, a loading unit, and a loading unit detection system. The adapter is releasably coupled to the handle. The loading unit is releasably coupled to the adapter. The loading unit detection system is configured to detect coupling of the loading unit to the adapter. The loading unit detection system includes a magnet and a GMR IC. The magnet is supported on the loading unit and translates relative to the elongate member as the loading unit is coupled to the adapter. The GMR IC is embedded within the adapter and is configured to output a differential voltage in response to a magnetic field produced by the magnet of the loading unit. The differential voltage produced by the GMR IC is indicative of the position of the loading unit within the elongate member.

In aspects, the adapter defines a receiver and the loading unit includes a connector. The connector may be received within the receiver to releasably couple the loading unit to the adapter. The magnet may be embedded within the connector of the loading unit. The GMR IC may be embedded within the receiver of the adapter.

In some aspects, the surgical instrument includes a microprocessor that is disposed within the handle. The microprocessor may receive a signal from the GMR IC that is indicative of the position of the loading unit relative to the adapter. The signal received by the microprocessor may be indicative of the type of loading unit.

Further, to the extent consistent, any of the aspects described herein may be used in conjunction with any or all of the other aspects described herein.

In a further aspect of the present disclosure, a surgical loading unit includes a pair of jaws, one of the jaws including a staple cartridge, and the other of the jaws having an anvil for forming staples. The loading unit has a loading unit detection system that includes a device for outputting an electromagnetic field. The loading unit is configured for use in a surgical system that includes a sensor for sensing the electromagnetic field, and communicating information about the loading unit to a microprocessor that is part of the surgical system. The loading unit can include an elongate member, and the detection system can be mounted on the elongate member, or the detection system can be mounted on the jaws. The detection system can be a giant magneto-resistance integrated circuit ("GMR IC"). The surgical system can include an elongate member defining a receiver and the loading unit can include a connector that is configured to be received within the receiver to releasably couple the loading unit to the elongate member. The magnet is supported on the connector. The magnet can be configured to translate relative to the elongate member as the connector is received within the receiver. The GMR IC is configured to output a differential voltage in response to a magnetic field produced by the magnet of the loading unit. The differential voltage can be indicative of the position of the loading unit within the receiver of the elongate member, indicative of characteristics of the loading unit, and can convey other information. The magnet may be embedded within the connector of the loading unit.

### BRIEF DESCRIPTION OF THE DRAWINGS

Various aspects of the present loading unit detection systems for surgical instruments are described hereinbelow with reference to the drawings, which are incorporated in and constitute a part of this specification, wherein:
FIG. 1 is a perspective view of a surgical instrument provided in accordance with the present disclosure including a handle, an adapter coupled to the handle, and a loading unit coupled to the adapter;
FIG. 2 is a perspective view of the surgical instrument of FIG. 1 with the loading unit separated from the adapter;
FIG. 3 is an enlarged view of the indicated area of detail of FIG. 2;
FIG. 4 is a side view of a distal portion of the adapter and a proximal portion of the loading unit of FIG. 3 with parts separated;
FIG. 5 is a side view of the distal portion of the adapter and the proximal portion of the loading unit of FIG. 3 with the loading unit coupled to the adapter;
FIG. 6 is a schematic of a detection circuit provided in accordance with the present disclosure; and
FIG. 7 is a schematic of another detection circuit provided in accordance with the present disclosure.

### DETAILED DESCRIPTION

Embodiments of the present loading unit detection systems for surgical instrumentsare now described in detail with reference to the drawings in which like reference numerals designate identical or corresponding elements in each of the several views. As used herein, the term "clinician" refers to a doctor, a nurse, or any other care provider and may include support personnel. Throughout this description, the term "proximal" refers to that portion of the device or component thereof that is closest to the clinician and the term "distal" refers to that portion of the device or component thereof that is farthest from the clinician.

Referring now to FIGS. 1-3, a surgical instrument 10 is provided in accordance with the present disclosure including a handle 20, an adaptor 30, and a loading unit 40. The adaptor 30 includes a proximal portion having a handle connector 32. The handle 20 defines an adaptor receiver 26 for receiving the handle connector 32 to releasably couple the adaptor 30 to the handle 20. The loading unit 40 has a proximal portion includes a loading unit connector 42. The adaptor 30 has a distal portion that defines a loading unit receiver 36 that is positioned to releasably couple the disposable loading unit 40 to the adaptor 30. The loading unit 40 includes an end effector assembly 140 including first and second jaw members 142, 144 that are moveable relative to one another and are configured to act on tissue. It is contemplated that the adapter 30 may be fixed to the handle 20 such that the adapter 30 defines an elongate portion extending from the handle 20.

An exemplary embodiment of a surgical instrument is disclosed in commonly owned U.S. Patent No. 9,055,943.

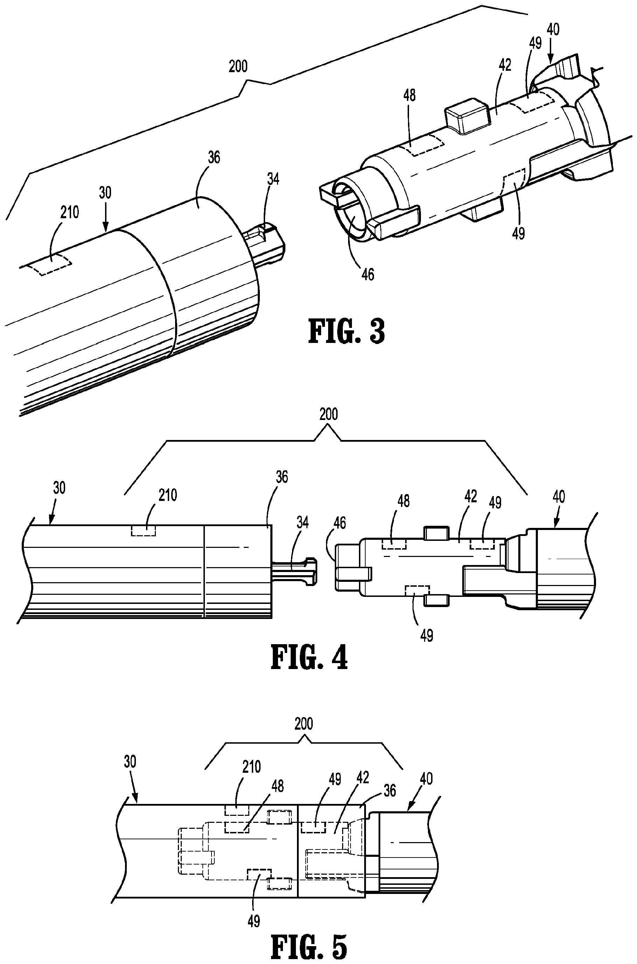

With particular reference to FIG. 3, the adapter 30 includes a translatable shaft 34 that extends distally through the loading unit receiver 36 and is configured to transfer mechanical energy from the adapter 30 to the loading unit 40 to actuate one or more functions of the end effector assembly 140 (FIG. 2), e.g., approximation and/or firing. The loading unit connector 42 of the loading unit 40 defines a shaft receiver 46 that receives the shaft 34 of the adapter 30. When the loading unit connector 42 is fully received within the loading unit receiver 36 the shaft 34 is coupled to the shaft receiver 46 such that translation of the shaft 34 actuates the end effector assembly 140.

With reference to FIGS. 3-5, the adapter 30 includes a contactless loading unit detection system 200 to determine the position of the loading unit connector 42 within the loading unit receiver 36. The loading unit detection system 200 includes a giant magneto-resistance integrated circuit (GMR IC) 210 embedded within a distal portion of the adapter 30 and the loading unit 40 includes a magnet 48 embedded within the loading unit connector 42. As detailed below, the GMR IC 210 is configured to output a differential voltage based upon the magnetic field induced in the GMR IC 210 by the magnet 48 of the loading unit 40. It will be appreciated that the differential voltage of the GMR IC 210 increases as a distance between the magnet 48 and the GMR IC 210 decreases.

In embodiments, the GMR IC 210 may be embedded within or adjacent the loading unit receiver 36 of the adapter 30 and/or the magnet 48 may be embedded within or adjacent the loading unit connector 42. By embedding both the GMR IC 210 and the magnet 48 within the loading unit receiver 36 and the loading unit connector 42, respectively, the loading unit detection system 200 does not include any exposed contacts. As such, fluid ingress into the loading unit detection system 200 is prevented. By preventing fluid ingress into the loading unit detection system 200, false indemnification of proper coupling of the loading unit 40 with the adapter 30 may be minimized.

The induced differential voltage of the GMR IC 210 is indicative of the position of the magnet 48 relative to the GMR IC 210 and thus, indicative of the position of the loading unit connector 42 of the loading unit 400 within the loading unit receiver 36 of the adapter 30. When the differential voltage of the GMR IC 210 reaches a predetermined threshold value, the loading unit connecter 42 is fully received within the loading unit receiver 36 (FIG. 5) such that the loading unit 40 is coupled to the adapter 30.

The GMR IC 210 may provide an analog or digital output indicative of the position of the loading unit connector 42 within the loading unit receiver 36. The handle 20 may include an indicator 28 configured to provide a visual indication of the position of the loading unit 40 within the adapter 30 and/or provide a visual indication when the loading unit 40 is coupled to the adapter 30.

In embodiments, the differential voltage of the GMR IC 210 may be used to identify the type of loading unit that is coupled to the adapter 30. For example, the loading unit 40 may include one or more additional magnets 49 that are/is configured to generate a unique magnetic field in the GMR IC 210 when the loading unit 40 is coupled to the adapter 30 such that the differential voltage of the GMR IC 210 is characteristic of the loading unit 40 that is coupled to the adapter 30. For example, in a linear stapling loading unit, the length of the staple line, staple size or sizes, characteristics such as whether the unit has a knife, dissection tip, buttress, etc., can be indicated. An indication that the unit has been used, is unused, or is within or beyond a certain prescribed number of uses, is contemplated.

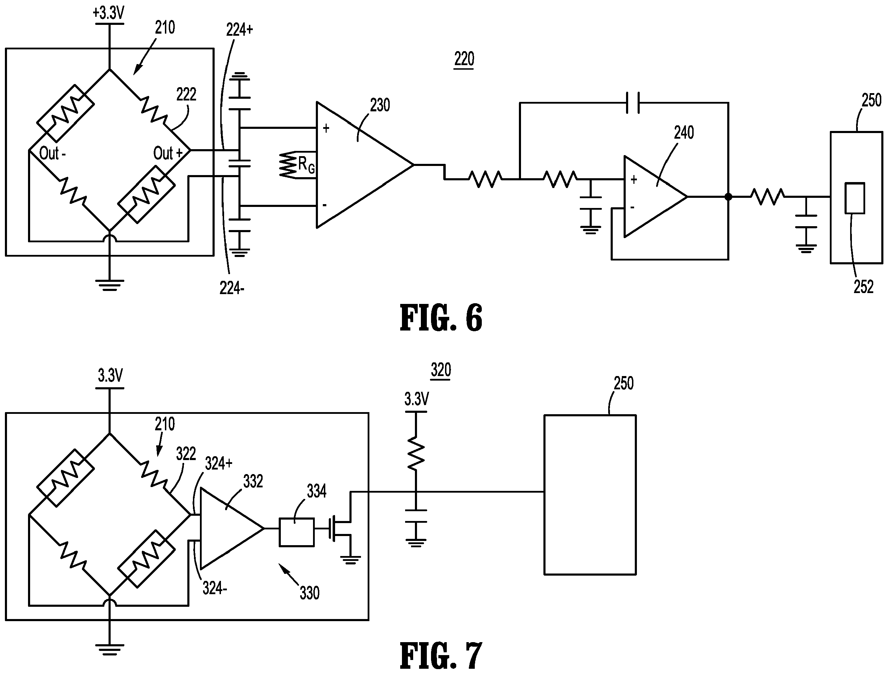

The differential voltage of the GMR IC 210 is received within a GMR circuit, e.g., GMR circuit 220 (FIG. 6) or GMR circuit 320 (FIG. 7) detailed below, and outputted to a microprocessor 250 (FIG. 1) disposed within the handle 20 of the surgical instrument 10. The microprocessor 250 analyzes the output of the GMR circuit to detect and/or identify a loading unit 40 is properly coupled to the adapter 30.

With reference to FIG. 6, a schematic of an illustrative GMR circuit 220 capable of detecting and identifying the presence and/or relative position of a loading unit, e.g., loading unit 40, is shown. The GMR circuit 220 includes the GMR IC 210 in the form of a partially shielded bridge 222 with positive and negative outputs 224+, 224-. The GMR IC 210 outputs a differential voltage signal based on a magnetic field induced in the GMR IC 210 by the magnets 48, 49 (FIG. 3). Specifically, as the magnetic field experienced by the GMR IC 210 varies, e.g., as the position of the magnets 48, 49 relative to the GMR IC 210 changes, the differential voltage of the output 224 varies. The outputs 224 of the GMR IC 210 are electrically coupled to an instrumentation amplifier 230 which outputs an amplified signal. The amplified signal may pass through a filter, e.g., low pass filter 240 before being transmitted to the microprocessor 250. The filtered signal of the low pass filter 240 may be transmitted to the analog to digital converter (ADC) 252 of the microprocessor 250 which analyzes the signal to identify the loading unit 40 based on a unique voltage signature and to determine when the loading unit 40 is coupled to the adapter 30. The microprocessor 250 may provide visual and/or audible indicia of the type of loading unit 40 and/or when the loading unit 40 is coupled to the adapter 30. The visual indicia may be provided on the display 28 (FIG. 1).

With reference to FIG. 7, a schematic of another illustrative GMR circuit 320 capable of detecting presence and/or relative position of a loading unit, e.g., loading unit 40, is shown. The GMR circuit 320 includes the GMR IC 210 in the form of a partially shielded bridge 322 with positive and negative outputs 324+, 324-. The GMR IC 210 outputs a differential voltage signal base on a magnetic field induced in the GMR IC 210 by the magnet 48. Specifically, as the magnetic field experienced by the GMR IC 210 varies, e.g., as the position of the magnet 48 relative to the GMR IC 210 changes, the differential voltage of the output 324 varies. The outputs 324 of the GMR IC 210 are electrically coupled to a comparator circuit 330 configured to detect when a signal from the GMR IC 210 exceeds a threshold value indicative of the position of the magnet 48 when the loading unit 40 is coupled to the adapter 30. The comparator circuit 330 may include an operational amplifier 332 and a latch 334 such that a connection signal is outputted from the latch 334 to the microprocessor 250 when the voltage of the GMR IC 210 is above the threshold value. It will be appreciated that no connection signal is outputted from the latch 334 when the voltage of the GMR IC 210 is below the threshold value. Alternatively, the latch 334 can be reversed such that the connection signal is outputted when the voltage of the GMR IC 210 is below the threshold value and is not outputted when the voltage of the GMR IC 210 is above the threshold value. The comparator circuit 330 may be integrated with the shielded bridge 322 to form an integrated GMR IC 210. The microprocessor may provide visual and/or audible indicia when the loading unit 40 is coupled to the adapter 30. The visual indicia may be provided on the display 28 (FIG. 1).

In embodiments according to the claimed invention, the adapter 30 includes a translating element (not shown) which is translated proximally as the loading unit connector 42 is received within the loading unit receiver 36. In such embodiments, the magnet 48 and/or magnet 49 may be embedded within the translating element and the GMR IC 210 may be embedded within the adapter 30 at a position to determine the position of the magnet 48. According to the invention, the entire detection system 200 is disposed within the adapter 30 and the GMR IC 210 may be positioned in a central or proximal portion of the adapter 30 remote from the loading unit receiver 36. According to the invention, the magnet 48 is embedded in the translating element and in some embodiments the magnets 49 are embedded within the loading unit connector 42 to induce a unique voltage in the GMR IC 210. For an exemplary translating element reference may be made to the detection link ring, the switch link ring, the switch link, the switch pin, or the switch button of the loading unit detection assembly of U.S. Patent Publication No. 2013/0324979.

It will be appreciated that the handle 20 and the adapter 30 may also include a contactless detection system similar to the contactless detection system 200 detailed above to detect when and/or the type of adapter 30 coupled to the handle 20.

In any of the embodiments disclosed herein, the microprocessor 250 can be provided in a hand-held housing, a remote console, or some other device. The loading unit can be configured to be used in a robotic surgical system. Also, the contactless detection system can include technologies other than magnetic, such as induction.

While several embodiments of the disclosure have been shown in the drawings, it is not intended that the disclosure be limited thereto, as it is intended that the disclosure be as broad in scope as the art will allow and that the specification be read likewise. Any combination of the above embodiments is also envisioned and forms part of the invention as long as the result falls within the scope of the appended claims. Therefore, the above description should not be construed as limiting, but merely as exemplifications of particular embodiments. Those skilled in the art will envision other modifications within the scope of the claims appended hereto.

## Claims

1. A surgical instrument comprising:
a handle (20);
an adapter (30) configured to releasably couple to the handle and including a translating element;
a loading unit (40) configured to releasably couple to a distal end portion of the adapter, the loading unit configured to translate the translating element of the adapter proximally as the loading unit is being coupled to the adapter which loading unit including an end effector assembly including a first and a second jaw member moveable relative to one another and configured to act on tissue;
wherein the translating element comprises a translatable shaft extending distally through the loading unit configured to transfer motion from the adapter to the loading unit to actuate one or more of the functions of the end effector, the translatable shaft being received in a shaft receiver of the loading unit such that translation of the shaft actuates the end effector;
a loading unit detection system comprising a magnet embedded in the translating element of the adapter and configured to translate with the translating element as the loading unit is coupled to the adapter; and
a GMR IC embedded within the adapter, the GMR IC configured to output a differential voltage in response to a magnetic field produced by the magnet, wherein the differential voltage produced by the GMR IC is indicative of a position of the loading unit relative to the adapter.

2. The surgical instrument according to claim 1, further comprising an operational amplifier and a latch, the operational amplifier configured to receive the differential voltage from the GMR IC and transmit an amplified output to the latch, the latch having a first state when the amplified output is below a threshold and a second state when the amplified output is above the threshold, preferably wherein the adapter defines a receiver and the loading unit includes a connector, the connector configured to be received within the receiver to releasably couple the loading unit to the adapter.

3. The surgical instrument according to claim 1 or 2, wherein the GMR IC is embedded within the receiver of the adapter.

4. The surgical instrument according to any one of claims 1 to 3, wherein the amplified output is below the threshold when the connector is in an uncoupled state with the receiver and above the threshold when the receiver is in a coupled state with the receiver.

5. The surgical instrument according to claim any one of claims 2 to 4, wherein the operation amplifier and latch are integrated with the GMR IC.

6. The surgical instrument according to any one of claims 2 to 5, further comprising a microprocessor disposed within the handle, the microprocessor configured to receive a signal from the GMR IC indicative of the position of the loading unit relative to the adapter preferably wherein the signal received by the microprocessor is indicative of the type of loading unit.

## Patentansprüche

1. Chirurgisches Instrument, umfassend:
einen Griff (20);
einen Adapter (30), der konfiguriert ist, um mit dem Griff lösbar gekoppelt zu werden und der ein Verschiebungselement einschließt;
eine Ladeeinheit (40), die konfiguriert ist, um mit einem distalen Endabschnitt des Adapters lösbar gekoppelt zu werden, wobei die Ladeeinheit konfiguriert ist, um das Verschiebungselement des Adapters proximal zu verschieben, wenn die Ladeeinheit mit dem Adapter gekoppelt wird, wobei die Ladeeinheit eine Endeffektoranordnung einschließt, die ein erstes und ein zweites Backenelement einschließt, die relativ zueinander beweglich sind und konfiguriert sind, um auf Gewebe zu wirken;
wobei das Verschiebungselement eine verschiebbare Welle umfasst, die sich durch die Ladeeinheit distal erstreckt und konfiguriert ist, um eine Bewegung von dem Adapter auf die Ladeeinheit zu übertragen, um eine oder mehrere der Funktionen des Endeffektors auszulösen, wobei die verschiebbare Welle in einem Wellenaufnehmer der Ladeeinheit derart aufgenommen ist, dass die Verschiebung der Welle den Endeffektor auslöst;
ein Ladeeinheitserkennungssystem, umfassend einen Magneten, der in das Verschiebungselement des Adapters eingebettet und konfiguriert ist, um sich mit dem Verschiebungselement zu verschieben, wenn die Ladeeinheit mit dem Adapter gekoppelt wird; und
einen GMR IC, der innerhalb des Adapters eingebettet ist, wobei der GMR IC konfiguriert ist, um als Reaktion auf ein Magnetfeld, das durch den Magneten erzeugt wird, eine Differenzspannung auszugeben, wobei die Differenzspannung, die durch den GMR IC erzeugt wird, eine Position der Ladeeinheit relativ zu dem Adapter anzeigt.

2. Chirurgisches Instrument nach Anspruch 1, ferner umfassend einen Operationsverstärker und ein Latch, wobei der Operationsverstärker konfiguriert ist, um die Differenzspannung von dem GMR IC aufzunehmen und eine verstärkte Ausgabe an das Latch zu übermitteln, wobei das Latch einen ersten Zustand, wenn die verstärkte Ausgabe unter einer Schwelle liegt, und einen zweiten Zustand aufweist, wenn die verstärkte Ausgabe über der Schwelle liegt, vorzugsweise wobei der Adapter einen Aufnehmer definiert und die Ladeeinheit einen Verbinder einschließt, wobei der Verbinder konfiguriert ist, um innerhalb des Aufnehmers aufgenommen zu werden, um die Ladeeinheit mit dem Adapter lösbar zu koppeln.

3. Chirurgisches Instrument nach Anspruch 1 oder 2, wobei der GMR-IC innerhalb des Aufnehmers des Adapters eingebettet ist.

4. Chirurgisches Instrument nach einem der Ansprüche 1 bis 3, wobei die verstärkte Ausgabe unter der Schwelle, wenn sich der Verbinder in einem nicht gekoppelten Zustand mit dem Aufnehmer befindet, und über der Schwelle liegt, wenn sich der Aufnehmer in einem gekoppelten Zustand mit dem Aufnehmer befindet.

5. Chirurgisches Instrument nach einem der Ansprüche 2 bis 4, wobei der Operationsverstärker und das Latch in den GMR-IC integriert sind.

6. Chirurgisches Instrument nach einem der Ansprüche 2 bis 5, ferner umfassend einen Mikroprozessor, der innerhalb des Griffs angeordnet ist, wobei der Mikroprozessor konfiguriert ist, um ein Signal von dem GMR IC aufzunehmen, das die Position der Ladeeinheit relativ zu dem Adapter anzeigt, vorzugsweise wobei das Signal, das durch den Mikroprozessor aufgenommen wird, die Art von Ladeeinheit anzeigt.

## Revendications

1. Instrument chirurgical comprenant :
une poignée (20) ;
un adaptateur (30) conçu pour s'accoupler de manière amovible à la poignée et comportant un élément de translation ;
une unité de chargement (40) conçue pour s'accoupler de manière amovible à une partie extrémité distale de l'adaptateur, l'unité de chargement étant conçue pour translater l'élément de translation de l'adaptateur de manière proximale tandis que l'unité de chargement est accouplée à l'adaptateur, ladite unité de chargement comportant un ensemble effecteur terminal comportant un premier et un second élément mâchoire mobiles l'un par rapport à l'autre et conçus pour agir sur du tissu ;
dans lequel l'élément de translation comprend une tige pouvant être translatée s'étendant de manière distale à travers l'unité de chargement conçue pour transférer un mouvement provenant de l'adaptateur vers l'unité de chargement afin d'actionner une ou plusieurs des fonctions de l'effecteur terminal, la tige pouvant être translatée étant reçue dans un récepteur pour tige de l'unité de chargement de sorte qu'une translation de la tige actionne l'effecteur terminal ;
un système de détection d'unité de chargement comprenant un aimant incorporé dans l'élément de translation de l'adaptateur et conçu pour translater avec l'élément de translation tandis que l'unité de chargement est accouplée à l'adaptateur ; et
un GMR IC incorporé à l'intérieur de l'adaptateur, le GMR IC étant configuré pour délivrer en sortie une tension différentielle en réponse à un champ magnétique produit par l'aimant, dans lequel la tension différentielle produite par le GMR IC indique une position de l'unité de chargement par rapport à l'adaptateur.

2. Instrument chirurgical selon la revendication 1, comprenant en outre un amplificateur opérationnel et un verrou, l'amplificateur opérationnel étant configuré pour recevoir la tension différentielle provenant du GMR IC et pour transmettre une sortie amplifiée au verrou, le verrou ayant un premier état lorsque la sortie amplifiée est inférieure à un seuil et un second état lorsque la sortie amplifiée est supérieure au seuil, de préférence dans lequel l'adaptateur définit un récepteur et l'unité de chargement comporte un raccord, le raccord étant conçu pour être reçu à l'intérieur du récepteur afin d'accoupler de manière amovible l'unité de chargement avec l'adaptateur.

3. Instrument chirurgical selon la revendication 1 ou 2, dans lequel le GMR IC est incorporé à l'intérieur du récepteur de l'adaptateur.

4. Instrument chirurgical selon l'une quelconque des revendications 1 à 3, dans lequel la sortie amplifiée est inférieure au seuil lorsque le raccord est dans un état désaccouplé avec le récepteur et est supérieure au seuil lorsque le récepteur est dans un état accouplé avec le récepteur.

5. Instrument chirurgical selon la revendication l'une quelconque des revendications 2 à 4, dans lequel l'amplificateur opérationnel et le verrou sont intégrés au GMR IC.

6. Instrument chirurgical selon l'une quelconque des revendications 2 à 5, comprenant en outre un microprocesseur disposé à l'intérieur de la poignée, le microprocesseur étant configuré pour recevoir un signal provenant du GMR IC indiquant la position de l'unité de chargement par rapport à l'adaptateur, de préférence dans lequel le signal reçu par le microprocesseur indique le type d'unité de chargement.
